# EUROPEAN PATENT APPLICATION

(11) **EP 1 747 782 A1**
(43) Date of publication of application: **31.01.2007**
(21) Application number: 05015645.4
(22) Date of filing: 19.07.2005
(51) Int. Cl.: A61K 31/685, A61K 31/20, A61P 21/00

(54) **Products for topical application comprising lysophospholipids and fatty acids**

(71) Applicant: Montecucco, Cesare, 35121 Padova (IT)
(72) Inventor: Caccin, Paola, 35030 Rubano (Padova) (IT); De Grandis, Domenico, 37060 Castel d'Azzano (Verona) (IT); Montecucco, Cesare, 35121 Padova (IT); Ravenni, Roberta, 45100 Rovigo (IT); Rigoni, Michela, 36100 Vicenza (IT); Rossetto, Ornella, 35141 Padova (IT); Schiavo, Giampietro, London NW3 2YP (GB)
(74) Representative: Zambardino, Umberto

(57) **Abstract**

It is described a product containing at least one lysophospholipid and at least one fatty acid, as a combined preparation for simultaneous, separate or sequential use, to be topically applied, in order to temporarily and reversibly inhibit a neuromuscular junction or the release of acetylcholine from autonomic nerve endings; this product can be used for treating hyperhidrosis conditions and for the cosmetic treatment of facial and other body wrinkles and, in general, human syndromes caused by the hyperactivity of peripheral nerve terminals.

## Description

### Field of application

The present invention refers, in general, to the pharmaceutical and cosmetic sector of industry.

The invention concerns, in particular a product for topical application, which is capable of causing a localized and temporary inhibition of peripheral synaptic terminals leading to blockade of neurotransmitter release at different types of nerve endings, including the neuromuscular junction (NMJ) and autonomic nerve endings that control glandular tissue and smooth muscles. Such a product can thus be used for treating a variety of pathological conditions caused by the hyperfunctionality of peripheral nerve terminals including palmar and axillary hyperhidrosis, and the pharmaco-cosmetic treatment of facial and neck wrinkles causing skin distension.

### State of the Known Art

Hyperhidrosis of the palms, soles of the feet and axillae is caused by excessive episodic sweating from the eccrine glands, as well as the apocrine glands in the case of axillae. This disorder is a cause of great distress for patients suffering therefrom.

Conventional therapies for hyperhidrosis (use of topical aluminum chloride salts, glutaraldehyde mixtures, anticholinergic drugs) are not free of inconveniences and are often unsuccessful.

Recently, the treatment with botulinum toxins for the reduction of hyperhidrosis has become quite popular. At present, hyperhidrosis is treated with commercially available preparations of botulinum toxin type A or B, which is injected intradermally at multiple sites causing an inhibition of the release from autonomic nerve terminals of the neurotransmitter acetylcholine which controls the release of sweat from the epithelial cells of the glands. This treatment, although being effective, is painful, requires the intervention of a skilled medical doctors and involves numerous intradermal injections in order to be effective.

The use of Botulinum A for treating hyperhidrosis by subcutaneous injection is disclosed in patent application WO 95/17904.

In this latter respect, attempts have been made to find out formulations for topically administering the botulinum toxin, without the need to resort to injection. For instance, patent application DE 19852981 discloses the topical administration of botulinum toxin in a DMSO solution, for treating hyperhidrosis. However, it is questionable that proteins as big as botulinum toxin might be absorbed to a significant extent and in any case formulations containing DMSO are not desirable because DMSO can irritate the skin.

Botulinum toxins have become very popular also in another field, namely the cosmetic field, for the treatment of facial wrinkles, in particular glabellar frown lines.

The theory underlying the pharmaco-cosmetic use of botulinum toxin is that contractile muscle fibers, which are under the direct control of the neural motor influx, play an important role in the formation of wrinkles, particularly glabellar lines. The local inhibition of the motor contraction by botulinum toxin minimizes wrinkles and exerts a smoothing effect on the skin.

However, this treatment is not free of inconveniences. For example, botulinum toxin may diffuse from the injected muscle to adjacent muscles and the resulting muscle paralysis may cause double vision or ptosis. In addition, the treatment causes frequently a decrease of facial expression. Moreover, it has been observed that some patients, after an initial benefit from this treatment, later became insensitive to it. This could be due to the development of antibodies against the toxin. Another significant drawback for botulinum toxin treatment lies in that the toxin has to be delivered by subcutaneous injections, performed by a skilled medical practitioner.

A method for topically administering the botulinum toxin without having to resort to injections is disclosed in patent application US 2005/0074461. This method involves a preliminary step of a mechanical disruption of the stratum corneum ((e.g. by abrasively removing it or by applying to the skin ultrasound at a certain frequency or electrical current etc.) and a subsequent step of applying botulinum toxin in the area that has had the stratum corneum disrupted.

This method is, however, apparently not suitable for being autonomously carried out by a subject in need for cosmetic treatment.

In light of the inconveniences of the known products and methods based on a temporary blockade of the NMJ or of the release of acetylcholine from autonomic nerve endings that control glandular tissue and smooth muscle, induced by the botulinum toxin, there is a need for new products that might exert such a blocking effect without the drawbacks of the known product, and thus provide for an improved treatment of conditions like hyperhidrosis and facial wrinkles.

### SUMMARY OF THE INVENTION

The problem underlying the present invention has, therefore, been that of providing new products for topical application onto the intact human skin, capable of bringing about a localized inhibition of nerve terminals in general - this includes, but not solely, the motoneurons terminals and the autonomic nerve endings controlling glandular tissue - while being topically administrable onto intact human skin.

Such a problem has been solved, according to the invention, by a product containing at least one lysophospholipid and at least one fatty acid, as a combined preparation for simultaneous, separate or sequential use, to be topically applied, in order to bring about a localized, temporary, specific and reversible inhibition of peripheral nerve terminals of any kind to, for instance, temporarily block a neuromuscular junction or impair the release of acetylcholine from autonomic nerve endings.

Such a product is useful for therapeutically treating hyperhidrosis conditions, in particular hyperhidrosis of palms, soles of the feet and axillae, for the cosmetic treatment of facial wrinkles, and in all those conditions which will benefit from the relaxation of superficial human muscles in general. The present application is also valid to effectively lower the smelling associated to excessive sweating and it can be included in anti axillary smelling products to increase their efficacy.

The product according to the invention can be a composition for topical application in the form of an ointment, paste, cream, fluid emulsion, solution, lotion, gel, microemulsion, vesicular dispersion containing ionic and non-ionic lipids, and it will contain at least one lysophospholipid, at least one fatty acid and a pharmaceutically or cosmetically acceptable vehicle.

Said at least one lysophospholipid (LysoPL) and said at least one fatty acid (FA) are preferably in a molar ratio ranging from 10:1 to 1: 10, conveniently 3:7 to 7:3 and most preferably in substantially equimolar quantities.

A lysophospholipid (LysoPL) is a compound of general formula R-O-CH₂-CH(OH)-CH₂-O-PO(OH)-OR', wherein R is an acyl residue of a fatty acid or a hydrocarbon chain, optionally containing -OH groups, and R' is any of the organic groups present in phospholipids head groups found in eukaryotic or prokaryotic cells (1,2).

R is preferably a C₈-C₂₂ acyl from both saturated and unsaturated fatty acid or a C₈-C₂₂ saturated or unsaturated hydrocarbon chain, optionally containing -OH groups.

These lysoPL may derive from natural sources and contain a mixture of acyl groups from fatty acids linked to position 1 of the glycerol moiety.

Preferred lysophospholipids are those selected among the group comprising lysophosphatidylcholine (LysoPC), lysophosphatidylserine (LysoPS), lysophosphatidyglycerol (LysoPG), lysophosphatidylethanolamine (LysoPE), lysophosphatidic acid (lysoPA).

FAs particularly suitable for the present invention are both saturated and unsaturated FAs having from 8 to 22 carbon atoms. Particularly preferred are saturated and unsaturated fatty acids having 12 to 18 C atoms, in particular myristic, palmitic, stearic, oleic, linoleic, linolenic, doxohexanoic acids and mixtures thereof.

The at least one lysophospholipid is generally contained in the composition of the invention in an amount of 1.0 % to 30 % by weight of the total weight of the composition. The same amount applies to the at least one fatty acid.

Preferably the lysophospholipids and the fatty acids are contained in the composition of the invention in an amount of 5.0 % to 15.0 % and 3.0 to 12.0 % by weight of the total weight of the composition respectively, and, most preferably, the lysophospholipids are contained in an amount of about 8% and the fatty acids in an amount of about 5% of the total weight of the composition.

The composition of the carrier may be any of those described in current textbooks of Pharmacy and Cosmetics for semi-solid preparations for cutaneous application, following the indications of the European Pharmacopoeia (3). In addition, the compositions according to the present invention may contain conventional adjuvants and additives, such as gelling agents, antioxidants, preservatives, solvents, perfumes, fillers, colorants, sun filters etc. They may also contain further active substances, such as e.g. vitamins.

In addition to the treatment and prevention of facial and neck wrinkles, and of hyperhidrosis, other possible applications of the product according to the invention include involuntary facial muscle spasms, synkinesis, blepharospasm and other eyelid spasms.

The characteristics and advantages of the present invention will be further appreciated with reference to the accompanying drawings and to the embodiments reported here below just by way of illustration and not of limitation.

### DETAILED DESCRIPTION OF THE INVENTION

The Applicants arrived at the results of the present invention in the course of their studies on the mechanism of action of snake presynaptic PLA2 neurotoxins. These presynaptic neurotoxins are major components of the venom of many poisonous snakes. Starting from: a) the established fact that these neurotoxins are endowed with a phospholipase A2 (PLA2) activity and that such activity leads to the production of LysoPLs and FAs following the hydrolysis of the fatty acid ester bond in position 2 of phospholipids (4); b) the fact that lysophospholipids and fatty acids in the appropriate membrane localization favor membrane fusion and inhibit membrane fission (5), and that c) such biological activities would lead to the depletion of synaptic vesicles from nerve terminals, as the one observed by electron microscopy upon exposure to the snake presynaptic PLA2 neurotoxins (6,7), the Applicants decided to test the effect of a mixture of lysophospholipids and fatty acids on the neuromuscular junction both in an ex-vivo preparation and in an in-vivo system. The results obtained are briefly described here below.

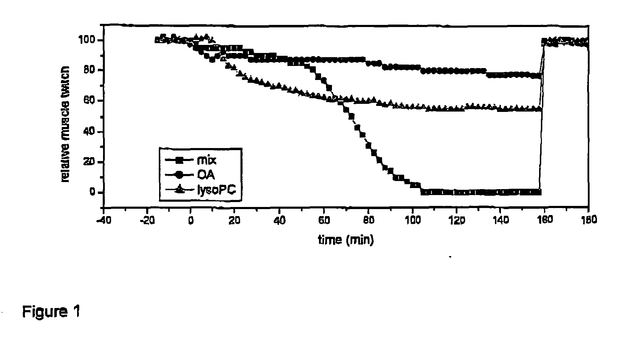

Fig. 1 shows the progressive and rapid inhibition caused by a 150 micromolar and equimolar mixture of oleic acid (OA) and 1-myristoyllysoPC (LysoPC). Similar data were obtained with other lysoPLs varying with respect to R and/or with respect to R'. At the same time, the figure illustrates the very important point that a lysoPC alone or OA molecule alone is unable to elicit a substantial inhibition. In other words, the two molecules act in a synergistic mode and this is at the very basis of the present patent.

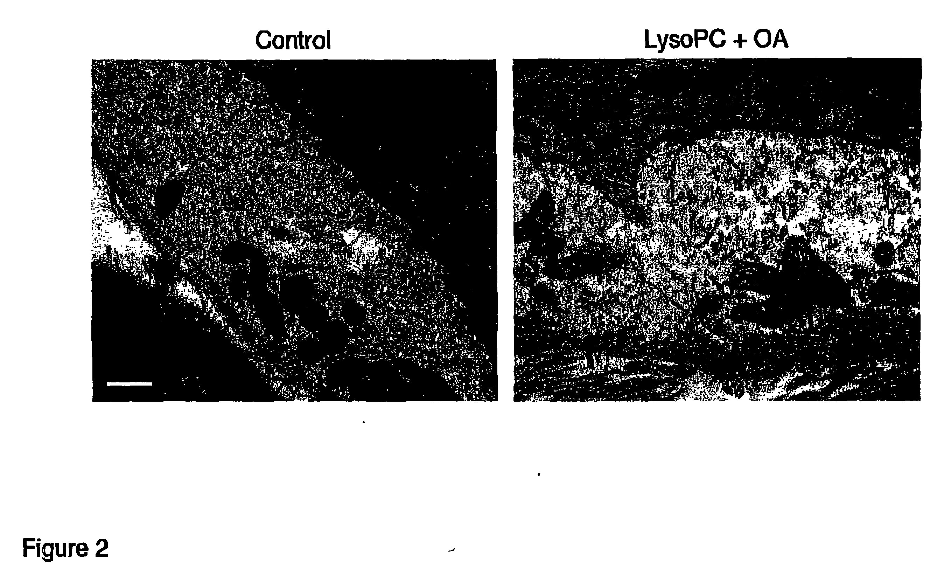

The right panel of fig. 2 shows an electron micrograph of a mouse phrenic nerve-hemidiaphragm neuromuscular junction paralysed by the addition of an equimolar mixture of oleic acid and 1-myristoyllysophosphatidylcholine, whilst the left panel of fig. 2 reports the controlateral, untreated NMJ. This figure shows that the lipid mixture inhibits the nerve terminal by inducing depletion of the synaptic vesicles containing the neurotransmitter, while leaving the muscle unaffected.

The nerve ending inhibiting activity of a mixture of a LysoPL and of FA can be explained on the basis of the biophysical theory of hemifusion intermediate in membrane fusion and fission (5). LysoPLs and FAs partition effectively into the presynaptic cell membrane and generate a membrane configuration which promotes synaptic vesicle exocytosis and inhibits their recycling. This leads to a swelling of the nerve terminal with depletion of the synaptic vesicles, as the one shown in the right panel of fig. 2, with inhibition of the nerve terminal activity. This inhibition is reversible upon removal of the lipid mixture by washings with a albumin in vitro, as it is shown in fig. 1 by the upward deflection at time 160 minutes, which follows the addition of albumin to the bath. In vivo, the removal of the LysoPLs and FAs is expected to be carried out by lipid metabolism and by the washing effect carried out by the tissue circulating fluids, leading to the reversal of the inhibitory effect seen on the human EDB muscles of volunteers, described here after.

The effect of an equimolar mixture of LysoPC and FA was also tested on human volunteers to assay the possibility that it elicits muscle relaxation. The lipid mixture was tested on the foot Extensor Digitorum Brevis (EDB) muscle, which has been validated for the assay of the NMJ paralysing activity of botulinum toxin (8). A cream composed of 1-myristoylphosphatidylcholine (7.7 %) and oleic acid (5 %) in a standard eudermic emulsion for galenic preparations was prepared following a standard protocol (3) and applied three times a day with gentle movements to the skin area overlaying the EDB of one foot, while the other foot was treated in the same way with the standard eudermic emulsion alone. A reduction ranging between 30 and 40 % in the compound muscular action potential amplitude was found after three days in a group of human volunteers and a similar decrease was found after seven days of treatment. After suspension from the treatment, the inhibited EDB muscle rapidly regained its entire contractile activity showing the complete reversibility of the induced effect. No side effects were noticed. Although preliminary, this result provides a clear proof of principle that the product according to the invention is capable of inhibiting the human neuromuscular junction in vivo, in all likelihood by inhibiting the release of acetylcholine from the motoneuron as it has been described above in fig. 1 for the ex-vivo neuromuscular junction preparation.

The product according to the invention is typically in the form of a composition for topical application, including a LysoPL or a mixture of LysoPLs, a FA or a mixture of FAs, and a pharmaceutically or cosmetically acceptable vehicle.

It is however also envisaged that the product according to the invention includes two separate formulations for topical use that can be applied the one immediately after the other or with a time interval between the two applications. In such a case, one of the two formulations will include at least one LysoPL and a pharmaceutically or cosmetically acceptable vehicle and the other one will include at least one FAs and a pharmaceutically or cosmetically acceptable vehicle.

Suitable vehicles for all of the above-mentioned compositions include all those excipients, solvents, diluents that are conventionally used in the pharmaceutical and cosmetic fields (3). Both components of the product according to the invention, i.e. lysoPLs and FAs, are amphiphilic, and thus they can be easily incorporated into hydrophobic vehicles to yield ointments and pastes, as well as into hydrophilic vehicles, thus providing for creams and emulsions (both W/O and O/W), with the optional addition of emulsifying agents.

Some examples of formulations according to the present invention are given here below.

### Example 1

### Ointment

| | |
|---|---|
| 1-Myristoyl-sn-glycero-3-phosphocholine | 7.7 % |
| Oleic acid | 5.0 % |
| Vaseline | 58.3 % |
| Cetostearyl alcohol | 23% |
| Cetomacrogol | 6% |

This ointment is prepared by heating the components together until they melt and agitating until cooling has occurred.

### Example 2

### W/O Cream

| | |
|---|---|
| 1-Myristoyl-sn-glycero-3-phosphocholine | 8 % |
| Oleic acid | 5.5 % |
| Vaseline | 37% |
| Almond oil | 3% |
| Cetostearyl alcohol | 17% |
| Cetomacrogol (1000) | 4.5% |
| Purified water | 25% |

The cream is prepared by melting the solid components in the vaseline and adding freshly boiled water at the same temperature, agitating slowly until cooling has occurred and reintegrating any evaporated water.

### Example 3

### Hydrophobic gel

| | |
|---|---|
| 1-Myristoyl-sn-glycero-3-phosphocholine | 8% |
| Oleic acid | 5 % |
| Almond Oil | 3 % |
| Vaseline | 17% |
| Cyclomethicone | 54% |
| Dimethiconol | 13% |

The gel is prepared by dispersing the 1-myristoyl-sn-glycero-3-phosphocholine, the oleic acid and the vaseline in a preformed mixture of cyclomethicone and dimethiconol.

### References:

1. Gurr M.I., Frayn K.N. & Harwood, J.L. (2001) Lipid Biochemistry Fifth Edition, Blackwell Publishing Ltd.
2. Berg, J.M., J.L. Tymoczko, and L. Stryer, Biochemistry. 5th ed. 2002, New York: W.H. Freeman.
3. European Pharmacopoeia 5.0 (2005) Semi-solid preparations for cutaneous application. Section 01/2005-0132.
4. Kini, R.M. Editor (1997). Venom Phospholipase A2 enzymes: structure, function and mechanism. Chichester, UK: Wiley.
5. Chernomordik, L.V. and Kozlov, M. M. (2003). Protein-lipid interplay in fusion and fission of biological membranes. Annu. Rev. Biochem. 72, 175-207
6. Cull-Candy, S.G., Fohlman, J., Gustavsson, D., Lullmann-Rauch, R., & Thesleff, S. (1976). The effects of taipoxin and notexin on the function and fine structure of the murine neuromuscular junction. Neuroscience 1, 175-180.
7. Montecucco, C., and Rossetto, O. (2000). How do presynaptic PLA2 neurotoxins block nerve terminals? Trends Biochem. Sci. 25, 266-270
8. Sloop R.R., Escutin R.O., Matus J.A., Cole B.A. & Peterson G.W. (1996). Dose-response curve of human extensor digitorum brevis muscle function to i.m. injected botulinum toxin type A. Neurology 46, 1382-1386.

## Claims

1. A product containing at least one lysophospholipid and at least one fatty acid, as a combined preparation for simultaneous, separate or sequential use, to be topically applied, in order to cause a localized inhibition of peripheral nerve terminals, leading to muscle relaxation and/or inhibition of sweat production.

2. A product according to claim 1, which is a composition for topical application containing at least one lysophospholipid, at least one fatty acid and a pharmaceutically or cosmetically acceptable vehicle.

3. A product according to claim 2, wherein said at least one lysophospholipid and said at least one fatty acid are in a molar ratio ranging from 10:1 to 1;10, preferably 3:7 to 7:3.

4. A product according to claim 3, wherein said at least one lysophospholipid and said at least one fatty acid are in substantially equimolar quantities.

5. A product according to any one of the preceding claims, wherein said at least one lysophospholipid is a compound of general formula R-O-CH₂-CH(OH)-CH₂-O-PO(OH)-OR', wherein R is an acyl residue of a fatty acid or a hydrocarbon chain, optionally containing -OH groups, and R' is any of the organic groups constituting the phospholipids head groups present in eukaryotic or prokeuyotic cells, the lysophospholipid being preferably selected among the group comprising lysophosphatidylcholine (LysoPC), lysophosphatidylserine (LysoPS), lysophosphatidylglycerol (LysoPG), lysophosphatidylethanolamine (LysoPE), and lysophosphatidic acid (LysvPA),

6. A product according to claim 5, wherein R is a C₈-C₂₂ acyl from both saturated and unsaturated fatty acids or a C₈-C₂₂ saturated or unsaturated hydrocarbon chain, optionally containing -OH groups.

7. A product according to any one of the preceding claims, wherein said at least one fatty acid is selected among saturated and unsaturated fatty acids having 8 to 22, preferably 12 to 18, carbon atoms and it is preferably selected among myristic, palmitic, stearic, oleic, linoleic, linolenic, doxohexanoic acids and mixtures thereof.

8. A product according to any one of claims 2 to 4, wherein said at least one lysophospholipid is contained in an amount of 1.0% to 30%, preferably 5 to 15%, by weight of the total weight of the composition, and said at least one fatty acid is contained in an amount of 1.0% to 30%, preferably 3 to 12%, by weight of the total weight of the composition.

9. A product according to any one of the preceding claims, which is a medicament for treating any pathological conditions deriving from an hyperactivity of peripheral nerve terminals, including hyperhidrosis.

10. A product according to any one of claims 1 to 8, which is used for the cosmetic treatment of facial and neck wrinkles.

11. Use of at least one lysophospholipid in combination with at least one fatty acid for the cosmetic treatment of human skin.

12. Use of at least one lysophospholipid and at least one fatty acid in the manufacture of a medicament for the treatment of hyperhidrosis.
